(19) Europäisches Patentamt
European Patent Office
Office européen des brevets

(11) Veröffentlichungsnummer : **0 413 667 B1**

(12) # EUROPÄISCHE PATENTSCHRIFT

(45) Veröffentlichungstag der Patentschrift :
**29.09.93 Patentblatt 93/39**

(51) Int. Cl.$^5$ : **C07C 225/16,** C07C 215/30, C07C 225/20, C07C 221/00, C07C 213/00, A61K 31/135

(21) Anmeldenummer : **90810608.1**

(22) Anmeldetag : **14.08.90**

(54) **Halogenalkylphenyl-Alkohole, -Ketone und deren Hydrate.**

(30) Priorität : **16.08.89 DE 3927049**

(43) Veröffentlichungstag der Anmeldung :
**20.02.91 Patentblatt 91/08**

(45) Bekanntmachung des Hinweises auf die Patenterteilung :
**29.09.93 Patentblatt 93/39**

(84) Benannte Vertragsstaaten :
**AT BE CH DE DK ES FR GB GR IT LI LU NL SE**

(56) Entgegenhaltungen :
**EP-A- 0 013 592**
**DE-A- 1 934 130**

(73) Patentinhaber : **SANDOZ LTD.**
**Lichtstrasse 35**
**CH-4002 Basel (CH)**
(84) **BE CH DK ES FR GB GR IT LI LU NL SE**
Patentinhaber : **SANDOZ-PATENT-GMBH**
**Humboldtstrasse 3**
**D-79539 Lörrach (DE)**
(84) **DE**
Patentinhaber : **SANDOZ-ERFINDUNGEN Verwaltungsgesellschaft m.b.H.**
**Brunner Strasse 59**
**A-1230 Wien (AT)**
(84) **AT**

(72) Erfinder : **Cottens, Sylvain**
**In den Reben 12**
**CH-4108 Witterswil (CH)**

EP 0 413 667 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue Halogenalkylphenyl-Alkohole, -Ketone und deren Hydrate, ihre Herstellung und Anwendung bei der therapeutischen Behandlung.

Diese im folgenden als neue Verbindungen bezeichneten Substanzen entsprechen der Formel I,

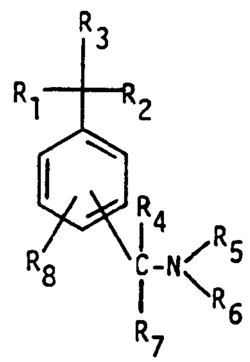

I

worin

| | |
|---|---|
| $R_1$ | für Wasserstoff oder Hydroxy steht, |
| $R_2$ | für Hydroxy steht oder mit $R_1$ eine Oxogruppe bildet, |
| $R_3$ | durch 1 bis 5 Halogenatome substituiertes Alkyl mit 1 bis 4 Kohlenstoffatomen bedeutet, |
| $R_4$ | für Wasserstoff oder Alkyl mit 1 bis 4 Kohlenstoffatomen steht, |
| $R_5$ und $R_5$, | unabhängig voneinander, je Alkyl mit 1 bis 4 Kohlenstoffatomen bedeuten, |
| $R_7$ | für Alkyl mit 1 bis 4 Kohlenstoffatomen steht, und |
| $R_5$ | Wasserstoff oder Alkyl mit 1 bis 4 Kohlenstoffatomen bedeutet, oder mit dem Dialkylaminoalkylrest in ortho-Stellung steht und mit $R_7$ eine $-(CH_2)_n$-Gruppe, worin n für 2, 3 oder 4 steht, bildet, |

in Form der freien Basen oder von Säureadditionssalzen.

Die neuen Verbindungen können je nach den vorhandenen Substituenten asymmetrische Kohlenstoffatome aufweisen. Die Erfindung beinhaltet alle daraus entstandenen Stereomere sowie ihre Gemische, beispielsweise die Racemate der Enantiomere.

Die in den neuen Verbindungen enthaltenen, vorstehend definierten Alkylgruppen enthalten vorzugsweise 1 oder 2 Kohlenstoffatome und stellen insbesondere Methyl dar.

Halogen im Substituent $R_3$ steht für Fluor, Chlor, Brom oder Jod, insbesondere für Fluor oder Chlor. $R_3$ steht z.B. für Difluormethyl, Trifluormethyl, Chlordifluormethyl oder Pentafluorethyl.

$R_4$ steht vorzugsweise für Wasserstoff.

Bevorzugt bedeutet $R_8$ Wasserstoff. Die zwei übrigen Substituenten auf dem Phenyl stehen vorzugsweise in meta-Stellung.

Bilden $R_7$ und $R_5$ zusammen eine $-(CH_2)_n$-Gruppe, so steht n vorzugsweise für 3.

Die Erfindung umfasst z.B. eine Gruppe von Verbindungen der Formel I, worin

| | |
|---|---|
| $R_1$ und $R_2$ | je für Hydroxy stehen oder zusammen eine Oxogruppe bilden, |
| $R_3$ | Difluormethyl, Trifluormethyl, Chlordifluormethyl oder Pentafluorethyl bedeutet, |
| $R_4$, $R_5$ und $R_5$ | obige Bedeutung besitzen, |
| $R_7$ | für Methyl steht, und |
| $R_5$ | Wasserstoff bedeutet, |

in Form der freien Basen oder von Säureadditionssalzen.

Es werden insbesondere jene Verbindungen der Formel I umfasst, worin $R_1$ und $R_2$ je für Hydroxy stehen, jene worin $R_3$ für Trifluormethyl steht, jene worin $R_4$ für Wasserstoff steht und $R_5$, $R_5$ und $R_7$ je für Methyl stehen, und jene worin $R_8$ für Wasserstoff steht und die zwei übrigen Substituenten auf dem Phenyl in meta-Stellung stehen, in Form der freien Basen oder von Säureadditionssalzen.

Die bevorzugte Verbindung ist das S-1-{3-[1-(Dimethylamino)ethyl]phenyl}-2,2,2-trifluor-1,1-ethandiol in Form der freien Base oder eines Säureadditionssalzes.

Erfindungsgemäss gelangt man zu den neuen Verbindungen und ihren Säureadditionssalzen, indem man

a) zur Herstellung der Verbindungen der Formel Ia

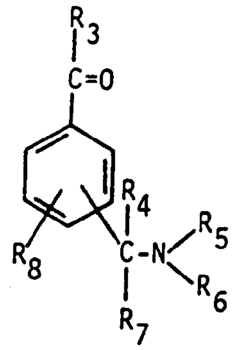

Ia

worin $R_3$ bis $R_8$ obige Bedeutung besitzen, in Verbindungen der Formel II,

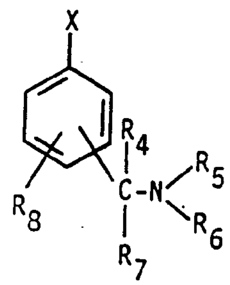

II

worin $R_4$ bis $R_8$ obige Bedeutung besitzen und X für Chlor, Brom oder Jod steht, das Halogen durch einen Rest $-COR_3$ substituiert, oder

b) zur Herstellung von Verbindungen der Formel Ib,

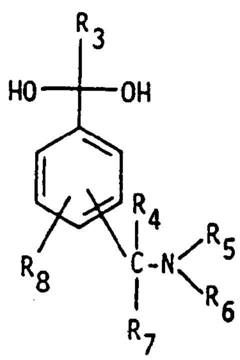

Ib

worin $R_3$ bis $R_8$ obige Bedeutung besitzen, Verbindungen der Formel Ia hydratisiert, oder

c) zur Herstellung von Verbindungen der Formel Ic,

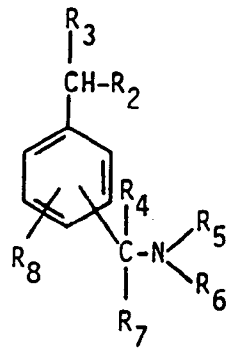

Ic

worin $R_2$ bis $R_8$ obige Bedeutung besitzen, Verbindungen der Formel Ia reduziert, und die erhaltenen Verbindungen der Formel I in Form der freien Basen oder in Säureadditionssalzform gewinnt.

In der Formel II steht X vorzugsweise für Brom.

Die Substitution des Halogens durch einen Rest $-COR_3$ gemäss Verfahren a) kann nach an sich bekannten Methoden erfolgen, beispielsweise durch Halogen-Metall-Austausch mit einem Organolithium wie n-Butyllithium oder mit Lithium und anschliessende Umsetzung mit einem reaktiven Derivat der Säure $R_3$-COOH, vorzugsweise einem Ester oder einem Amid. Der Halogen-Austausch durch Lithium wird vorzugsweise bei einer niederen Temperatur, z.B. zwischen - 100 und - 30 ° C durchgeführt, in einem aprotischen Lösungsmittel, vorzugsweise Tetrahydrofuran. Die Umsetzung mit dem Säurederivat erfolgt zweckmässigerweise bei der gleichen niederen Temperatur. Vorzugsweise lässt man einige Stunden reagieren und gibt dann dem Reaktionsgemisch Wasser zu.

Die Hydratisierung der Verbindungen der Formel Ia gemäss Verfahren b) kann auf an sich bekannte Weise erfolgen, vorzugsweise in Anwesenheit einer Säure, z.B. einer Mineralsäure wie Chlorwasserstoff oder Bromwasserstoff.

Die Reduktion der Verbindungen der Formel Ia gemäss Verfahren c) kann nach an sich bekannten Methoden erfolgen. Als Reduktionsmittel werden vorzugsweise Metallhydride, z.B. Lithiumaluminiumhydrid oder Natriumborhydrid verwendet.

Die Aufarbeitung der gemäss obigen Verfahren erhaltenen Reaktionsgemische und die Reinigung der so gewonnenen Verbindungen der Formel I kann nach an sich bekannten Methoden durchgeführt werden.

Die Enantiomerentrennung, falls gewünscht, kann nach an sich bekannten Methoden erfolgen, z.B. via vorübergehende Säureadditionssalzbildung mit optisch aktiven Säuren, z.B. (+)-[bzw. (-)]-Di-O,O'-p-toluoyl-D-(-)[bzw. L-(+)]-weinsäure, und fraktionierter Kristallisation der diastereoisomeren Säureadditionssalze.

Aus den freien Basen lassen sich in bekannter Weise Säureadditionssalze herstellen und umgekehrt.

Die Ausgangsverbindungen der Formel II können ausgehend von Verbindungen der Formel III,

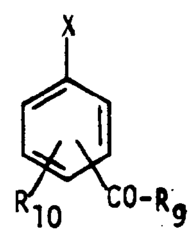

III

worin X obige Bedeutung besitzt, $R_9$ für Methyl steht und $R_{10}$ Wasserstoff bedeutet oder mit dem $R_9$-CO-Rest in ortho-Stellung steht und mit $R_9$ eine oben definierte $-(CH_2)_n-$Gruppe bildet, nach an sich bekannten Methoden, beispielsweise wie in Beispielen 1 und 8 beschrieben, hergestellt werden.

Soweit die Herstellung der Ausgangsprodukte nicht beschrieben wird, sind diese bekannt oder nach an sich bekannten Verfahren bzw. analog zu an sich bekannten Verfahren herstellbar.

Die Verbindungen der Formel I und ihre Säureadditionssalze mit physiologisch verträglichen Säuren, im folgenden als erfindungsgemässe Verbindungen bezeichnet, zeichnen sich durch pharmakologische Wirkung aus und sind deshalb verwendbar als Pharmazeutika.

Die erfindungsgemässen Verbindungen üben insbesondere eine selektive Hemmung der Acetylcholinesteraseaktivität in verschiedenen Regionen des Gehirns aus, die bei der Ratte ex vivo nach Verabreichung von Dosen zwischen 0,3 und 10,0 μmol/kg p.o. festgetellt wurde.

Die Acetylcholinesteraseaktivität wird entsprechend der durch Elman (Arch. Biochem. Biophys. 82, 70, 1959) beschriebenen spektrophotometrischen Methode gemessen. Das Rattenhirngewebe wird in kaltem Phosphatpuffer pH 7,3 (0,25 mM), enthaltend 0,1 % Triton X-100, homogenisiert. Nach der Zentrifugation werden Aliquots des Klarobenaufschwimmenden als Enzymquelle verwendet.

In diesem Versuch wird mit Enzym aus verschiedenen Hirnregionen eine signifikante Hemmung festgestellt, während Enzym aus peripheren Organen wenig beinflusst wird. Nach Verabreichung von S-1-{3-[1-(Dimethylamino)ethyl]phenyl}-2,2,2-trifluor-1,1-ethandiol, z.B., wird mit Enzym aus Cortex, Hippocampus, Striatum und Pons/Medulla eine starke Hemmung festgestellt, während Enzym aus dem Herz nicht beeinflusst wird.

Die erfindungsgemässen Verbindungen sind daher brauchbar als selektive Acetylcholinesterasehemmer, z.B. zur Behandlung der senilen Demenz, Alzheimer's Disease, Huntington's Chorea, tardiven Dyskinesien, Hyperkinesie, Manie, akuten Verwirrungszuständen, Down's Syndrom, Myasthenia gravis, Friedrich's Ataxie und Schmerzen.

Eine geeignete Tagesdosis liegt im Bereich von etwa 0,1 bis 50 mg der Substanz, geeignete Dosierungsformen für z.B. orale Anwendungen enthalten im allgemeinen ungefähr 0,025 bis 25 mg einer erfindungsgemässen Verbindung, neben festen oder flüssigen Trägersubstanzen oder Verdünnungsmitteln.

Als Heilmittel können die erfindungsgemässen Verbindungen allein oder in geeigneter Arzneiform mit pharmakologisch indifferenten Stoffen verabreicht werden.

Gegenstand der Erfindung sind auch pharmazeutische Zusammensetzungen, die die erfindungsgemässen Verbindungen als Wirkstoffe enthalten. Für ihre Herstellung können die in der Pharmazie gebräuchlichen Hilfs- und Trägerstoffe verwendet werden. Geeignete galenische Formen sind z.B. Tabletten, Kapseln und Tropflösungen.

Die nachfolgenden Beispiele erläutern die Erfindung. Temperaturen erfolgen in Celsiusgraden und sind unkorrigiert.

**BEISPIEL 1: 1-{3-[1(Dimethylamino)ethyl]phenyl}-2,2,2-trifluorethanon**

10 g 3-Brom-N,N,α-trimethylbenzenmethanamin werden in 80 ml Tetrahydrofuran gelöst und auf - 77 ° gekühlt. Dazu tropft man 30 ml (48 mMol) n-Butyllithium in Hexan und rührt das Reaktionsgemisch bei dieser Temperatur während zehn Minuten. Danach werden 8,0 g (47 mMol) Ethyltrifluoracetat zugegeben. Nach 2 Stunden wird das Reaktionsgemisch mit 10 ml Wasser versetzt und auf 600 ml Essigester gegossen. Die organische Phase wird mit Wasser gewaschen, getrocknet und schliesslich eingeengt. Nach Destillation des verbleibenden Oels erhält man das reine Trifluormethylketon; Kp: 75 - 80 °/ 5 mmHg; $^1$H-NMR (CDCl3, TMS) δ: 1,38 (d, J=7, 3H); 2,20 (s, 3H); 3,35 (q, J=7, 1H); 7,51 (t, J=8, 1H); 7,70 (d, J=8, 1H); 7,93 (d, J=8, 1H); 8,00 (s, 1H).

Das als Ausgangsmaterial verwendete 3-Brom-N,N,α-trimethylbenzenmethanamin kann wie folgt hergestellt werden:
60 g (0,30 Mol) 3-Bromacetophenon werden in 800 ml Ethanol gelöst und mit 250 ml Dimethylamin versetzt. Der pH der Lösung wird mit Essigsäure auf 6,5 eingestellt. Das Reaktionsgemisch wird dann auf 0 ° gekühlt und mit 19 g Natriumcyanborhydrid versetzt. Nach 2 Tagen Rühren bei Raumtemperatur engt man das Reaktionsgemisch ein. Der Rückstand wird mit 800 ml Wasser versetzt und angesäuert. Dann wird 2 x mit 400 ml Aether extrahiert, die wässrige Phase basisch gestellt und noch 3 x mit 400 ml Aether extrahiert. Die vereinigten organischen Auszüge werden getrocknet und eingeengt. Durch Destillation des Rückstandes erhält man das 3-Brom-N,N,α-trimethylbenzenmethanamin; Kp: 98 - 100 °/10 mmHg.

**BEISPIEL 2: 1-{3-[1-(Dimethylamino)ethyl]phenyl}-2,2,2-trifluor-1,1-ethandiol**

5 g der im Beispiel 1 erhaltenen Verbindung werden in 100 ml Aether gelöst und die Lösung wird mit einem Ueberschuss Chlorwasserstoff angesäuert. Nach Umkristallisation des ausgefallenen Feststoffes aus Aceton/Wasser erhält man das reine Hydrochlorid der Titelverbindung. Smp.: 137 - 139 °.

**BEISPIEL 3: S-1-{3-[1-(Dimethylamino)ethyl]phenyl}-2,2,2-trifluorethanon**

10,0 g racemisches 3-Brom-N,N,α-trimethylbenzenmethanamin und 10,1 g (+)-Di-O,O'-p-toluyl-D-weinsäure-monohydrat werden in 700 ml Aceton heiss gelöst. Das nach Abkühlen ausgefallene Salz wird abfiltriert und aus einem Gemisch aus 1 Liter Aceton und 25 ml Methanol umkristallisiert. Durch Verteilen des erhaltenen Kristallisats zwischen 10 %iger $Na_2CO_3$ und Aether erhält man das (-)-Enantiomer des 3-Brom-N,N,α-

trimethylbenzenmethanamins;
$[\alpha]_D^{20}$ = -41,1 ° (c = 1, MeOH).

Das (-)-3-Brom-N,N,α-trimethylbenzenmethanamin wird nach der im Beispiel 1 beschriebenen Methode in die Titelverbindung umgewandelt; $[\alpha]_D^{20}$ = - 44,8 ° (c =1, Aceton).

**BEISPIEL 4: S-1-{3-[1-(Dimethylamino)ethyl]phenyl]-2,2,2-trifluor-1,1-ethandiol**

Durch Behandeln von (-)-1-{3-[1-(dimethylamino)ethyl]phenyl}-2,2,2-trifluorethanon mit HCl in Aether, wie im Beispiel 2 beschrieben, erhält man das S-1-{3-[1-(Dimethylamino)ethyl]phenyl}-2,2,2-trifluor-1,1-ethandiol in Form des Hydrochlorids;
$[\alpha]_D^{20}$ = - 6,5 ° (c = 1, Wasser).

$^1$H-NMR (DMSO-$d_6$): δ = 1,65 (d, J=6, 3H); 2,48 (d, J=5, 3H); 2,71 (d, J=5, 3H); 4,5 - 4,6 (m, 1H); 7,52 (t, J=8, 1H); 7,1 - 7,2 (m, 5H); 11,1 (br.s, 1H).

MS (FAB): 264 ($MH^+$, 100 %); 219 (38 %).

Analog Beispiel 1 erhält man folgende Verbindungen (Beispiele 5 bis 9):

**BEISPIEL 5: 1-{3-[1-(Dimethylamino)ethyl]phenyl}-2,2-difluorethanon**

$^1$H-NMR ($CDCl_3$): δ = 1,39 (d, J=7, 3H); 2,20 (s, 6H); 3,36 (q, J=7, 1H); 6,33 (t, J=54, 1H); 7,50 (t, J=8, 1H); 7,67 (d, J=8, 1H); 8,0 (m, 2H).

MS: 227 (18%); 212 (100 %); 133 (75 %); 72 (95 %).

**BEISPIEL 6: 2-Chlor-1-{3-[1-(Dimethylamino)ethyl]phenyl}-2,2-difluorethanon**

$^1$H-NMR ($CDCl_3$): δ = 1,38 (d, J=7, 3H); 2,19 (s, 6H); 3,35 (q, J=7, 1H); 7.48 (t, J=8, 1H); 7,68 (d, J=8, 1H); 8,0 (m, 2H).

MS: 263 (2 %); 261 (5 %); 248 (18 %); 246 (52 %); 72 (100 %).

**BEISPIEL 7: 1-{3-[1-(Dimethylamino]ethyl]phenyl}-2,2,3,3,3-pentafluorpropanon**

$^1$H-NMR ($CDCl_3$): δ = 1,38 (d, J=7, 3H); 2,20 (s, 6H); 3,35 (q, J=7, 1H); 7,50 (t, J=8, 1H); 7,70 (d, 1H); 8,0 (m, J=8, 2H).

**BEISPIEL 8: 1-(5-Dimethylamino-5,6,7,8-tetrahydro-1-naphthalenyl)-2,2,2-trifluorethanon**

$^1$H-NMR ($CDCl_3$): δ = 1,6 - 1,7 (m, 2H); 1,9 - 2,1 (m, 2H); 2,25 (s, 6H); 2,9 (m, 2H); 3,7 - 3,8 (m, 1H); 7,32 (t, J=7, 1H); 7,71 (d, J=7, 1H); 8,03 (d, J=7, 1H).

MS: 271 (30 %); 242 (30 %); 226 (50 %); 157 (100 %).

Das Ausgangsmaterial kann wie folgt hergestellt werden:

8,5 g 5-Bromtetralon werden in 100 ml Ethanol gelöst und mit 25 ml einer 33 %igen Methylaminlösung in Ethanol versetzt. Das Gemisch wird auf 5 ° gekühlt und der pH auf 6 - 7 mit Essigsäure gestellt. Dann werden 2,5 g Natriumcyanborhydrid portionenweise zugegeben. Anschliessend rührt man 36 Stunden bei Raumtemperatur. Man dampft das Lösungsmittel ein und gibt den Rückstand auf 1 N NaOH. Die wässrige Phase wird 3 mal mit Aether extrahiert. Die vereinigten organischen Auszüge werden über $Na_2SO_4$ getrocknet und eingedampft. Das verbleibende Oel (8,7 g) wird in 100 ml Methanol gelöst und mit 6 ml einer 33 %igen wässrigen Formaldehydlösung versetzt. Das Gemisch wird auf 5 ° gekühlt und mit 2,8 g Natriumborhydrid versetzt. Nach 24 Stunden Rühren bei Raumtemperatur engt man das Reaktionsgemisch ein und gibt den Rückstand auf 1 N HCl. Die wässrige Phase wird 2 x mit Aether gewaschen. Dann wird der pH mit 30 %igem NaOH auf 10 gestellt und man extrahiert 3 x mit Aether. Die organischen Auszüge werden über $Na_2SO_4$ getrocknet und eingeengt. Man erhält das 5-Brom-N,N-dimethyl-1,2,3,4-tetrahydro-1-naphthalenylamin.

$^1$H-NMR ($CDCl_3$): δ = 1,6 (m, 2H); 1,9 (m, 1H); 2,1 (m, 1H); 2,24 (s, 6H); 2,6 (m, 1H); 2,8 (m, 1H); 3,7 (m, 1H); 7,03 (t, J=7, 1H); 7,40 (d, J=7, 1H); 7,62 (d, J=7, 1H).

**BEISPIEL 9: 1-(8-Dimethylamino-5,6,7,8-tetrahydro-2-naphthalenyl)-2,2,2-trifluorethanon**

$^1$H-NMR (CDCl3): δ = 1,6 -1,7 (m, 2H); 1,9 - 2,1 (m, 2H); 2,27 (s, 6H); 2,8 - 2,9 (m, 1H); 3,7 - 3,8 (m, 1H); 7,21 (d, J=7, 1H); 7,81 (d, J=7, 1H); 8,4 (s, 1H).

MS: 271 (50 %); 242 (95 %); 226 (75 %); 157 (100 %).

**BEISPIEL 10: 1-{3-[1-(Dimethylamino)ethyl]phenyl}-2,2,2-trifluorethanol**

30 mg des im Beispiel 3 erhaltenen Ketons werden in 6 ml Tetrahydrofuran gelöst und zu einer Suspension von 100 mg Lithiumaluminiumhydrid in 4 ml Tetrahydrofuran bei 0 ° zugetropft. Nach 5 Stunden Rühren bei Raumtemperatur tropft man 1 ml Methanol und 0,5 ml Wasser zu. Anschliessend filtriert man über Hyflo und dampft das Filtrat am Rotationsverdampfer zur Trockene ein. Man erhält 452 mg eines Diastereomerengemisches der Titelverbindung.

$^1$H-NMR (CDCl3): δ = 1,37 (d, J=6) und 1,38 (d, J=6) insgesamt 3H; 2,23 (s, 6H); 3,21 (g, J=6) und 3,36 (q, J=6) insgesamt 1H; 4,58 (q, J=6) und 4,85 (q, J=6) insgesamt 1H; 5,9 (br. s, 1H); 7,3 - 7,5 (m, 4H).

MS: 248 (100 %); 232 (25 %); 203 (40 %).

## Patentansprüche

**Patentansprüche für folgende Vertragsstaaten : AT, BE, CH, LI, DE, DK, FR, GB, IT, LU, NL, SE**

1. Eine Verbindung der Formel I,

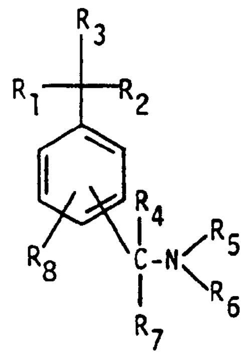

I

worin

| | |
|---|---|
| $R_1$ | für Wasserstoff oder Hydroxy steht, |
| $R_2$ | für Hydroxy steht oder mit $R_1$ eine Oxogruppe bildet, |
| $R_3$ | durch 1 bis 5 Halogenatome substituiertes Alkyl mit 1 bis 4 Kohlenstoffatomen bedeutet, |
| $R_4$ | für Wasserstoff oder Alkyl mit 1 bis 4 Kohlenstoffatomen steht, |
| $R_5$ und $R_6$, | unabhängig voneinander, je Alkyl mit 1 bis 4 Kohlenstoffatomen bedeuten, |
| $R_7$ | für Alkyl mit 1 bis 4 Kohlenstoffatomen steht, und |
| $R_8$ | Wasserstoff oder Alkyl mit 1 bis 4 Kohlenstoffatomen bedeutet, oder mit dem Dialkylaminoalkylrest in ortho-Stellung steht und mit $R_7$ eine $-(CH_2)_n-$Gruppe, worin n für 2, 3 oder 4 steht, bildet, |

in Form der freien Base oder eines Säureadditionssalzes.

2. Eine Verbindung gemäss Anspruch 1, worin

| | |
|---|---|
| $R_1$ und $R_2$ | je für Hydroxy stehen oder zusammen eine Oxogruppe bilden, |
| $R_3$ | Difluormethyl, Trifluormethyl, Chlordifluormethyl oder Pentafluorethyl bedeutet, |
| $R_4$, $R_5$ und $R_5$ | wie im Anspruch 1 definiert sind, |
| $R_7$ | für Methyl steht, und |
| $R_8$ | Wasserstoff bedeutet. |

3. Das S-1-{3-[1-(Dimethylamino)ethyl]phenyl}-2,2,2-trifluor-1,1-ethandiol in Form der freien Base oder eines Säureadditionssalzes.

4. Eine Verbindung gemäss Anspruch 1 aus der Gruppe, bestehend aus
   1-{3-[1-(Dimethylamino)ethyl]phenyl}-2,2,2-trifluorethanon,

1-{3-[1-(Dimethylamino)ethyl]phenyl}-2,2,2-trifluor-1,1-ethandiol,
S-1-{3-[1-(Dimethylamino)ethyl]phenyl}-2,2,2-trifluorethanon,
1-{3-[1-(Dimethylamino)ethyl]phenyl}-2,2-difluorethanon,
2-Chlor-1-{3-[1-(Dimethylamino)ethyl]phenyl}-2,2-difluorethanon,
1-{3-[1-(Dimethylamino)ethyl]phenyl}-2,2,3,3,3-pentafluorpropanon,
1-(5-Dimethylamino-5,6,7,8-tetrahydro-1-naphthalenyl)-2,2,2-trifluorethanon,
1-(8-Dimethylamino-5,6,7,8-tetrahydro-2-naphthalenyl)-2,2,2-trifluorethanon und
1-{3-[1-(Dimethylamino)ethyl]phenyl}-2,2,2-trifluorethanol,

in Form der freien Base oder eines Säureadditionssalzes.

**5.** Verfahren zur Herstellung einer Verbindung gemäss Anspruch 1, dadurch gekennzeichnet, dass man
a) zur Herstellung der Verbindungen der Formel I a,

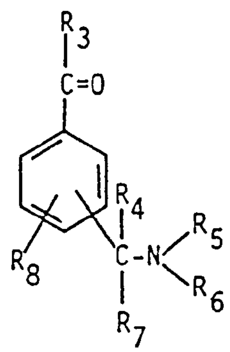

(I a)

worin $R_3$ bis $R_8$ wie im Anspruch 1 definiert sind, in Verbindungen der Formel II,

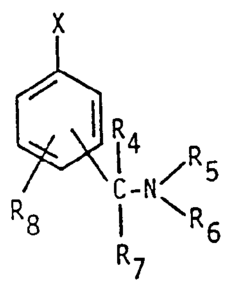

(II)

worin $R_4$ bis $R_8$ wie im Anspruch 1 definiert sind und X für Chlor, Brom oder Jod steht, das Halogen durch einen Rest $-COR_3$ substituiert, oder
b) zur Herstellung der Verbindungen der Formel I b,

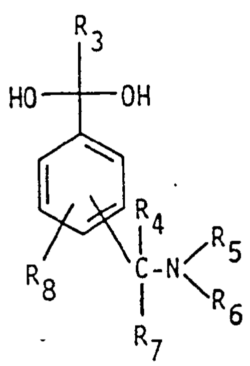

(I b)

worin $R_3$ bis $R_8$ wie im Anspruch 1 definiert sind, Verbindungen der Formel I a hydratisiert, oder
c) zur Herstellung von Verbindungen der Formel I c,

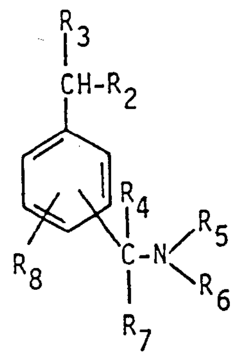

(I c)

worin $R_2$ bis $R_8$ wie im Anspruch 1 definiert sind, Verbindungen der Formel I a reduziert, und die erhaltenen Verbindungen der Formel I in Form der freien Basen oder in Säureadditionssalzform gewinnt.

**6.** Eine Verbindung gemäss einem der Ansprüche 1 bis 4, in freier oder pharmakologisch verträglicher Säureadditionssalzform, zur Anwendung als Pharmazeutikum.

**7.** Eine Verbindung gemäss einem der Ansprüche 1 bis 4, in freier oder pharmakologisch verträglicher Säureadditionssalzform, zur Behandlung der senilen Demenz, Alzheimer's Disease, Huntington's Chorea, tardiven Dyskinesien, Hyperkinesie, Manie, akuten Verwirrungszuständen, Down's Syndrom, Myasthenia gravis, Friedrich's Ataxie und Schmerzen.

**8.** Eine pharmazeutische Zusammensetzung, enthaltend eine Verbindung gemäss einem der Ansprüche 1 bis 4, in freier oder pharmazeutisch verträglicher Säureaddiditionssalzform.

**Patentansprüche für folgende Vertragsstaaten : ES, GR**

**1.** Verfahren zur Herstellung einer Verbindung der Formel I,

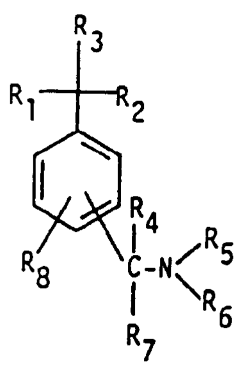

I

worin

| | |
|---|---|
| $R_1$ | für Wasserstoff oder Hydroxy steht, |
| $R_2$ | für Hydroxy steht oder mit $R_1$ eine Oxogruppe bildet, |
| $R_3$ | durch 1 bis 5 Halogenatome substituiertes Alkyl mit 1 bis 4 Kohlenstoffatomen bedeutet, |
| $R_4$ | für Wasserstoff oder Alkyl mit 1 bis 4 Kohlenstoffatomen steht, |
| $R_5$ und $R_6$, | unabhängig voneinander, je Alkyl mit 1 bis 4 Kohlenstoffatomen bedeuten, |
| $R_7$ | für Alkyl mit 1 bis 4 Kohlenstoffatomen steht, und |
| $R_8$ | Wasserstoff oder Alkyl mit 1 bis 4 Kohlenstoffatomen bedeutet, oder mit dem Dialkylaminoalkylrest in ortho-Stellung steht und mit $R_7$ eine $-(CH_2)_n-$Gruppe, worin n für 2, 3 oder 4 steht, bildet, |

in Form der freien Base oder eines Säureadditionssalzes, dadurch gekennzeichnet, dass man

a) zur Herstellung der Verbindungen der Formel Ia,

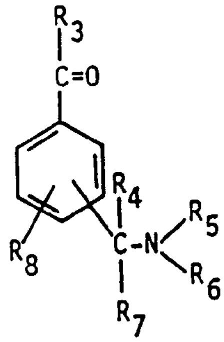

Ia

worin $R_3$ bis $R_8$ wie im Anspruch 1 definiert sind, in Verbindungen der Formel II,

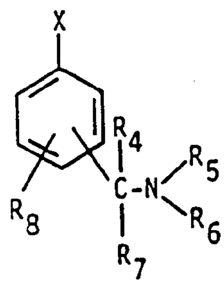

II

worin $R_4$ bis $R_8$ wie im Anspruch 1 definiert sind und X für Chlor, Brom oder Jod steht, das Halogen durch einen Rest $-COR_3$ substituiert, oder
b) zur Herstellung der Verbindungen der Formel Ib,

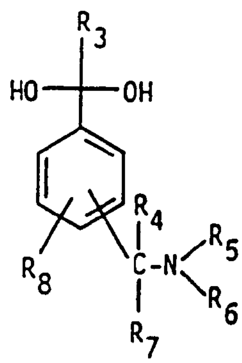

Ib

worin $R_3$ bis $R_8$ wie im Anspruch 1 definiert sind, Verbindungen der Formel Ia hydratisiert, oder
c) zur Herstellung von Verbindungen der Formel Ic,

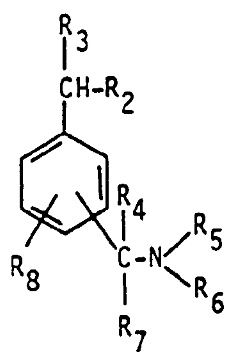

Ic

worin $R_2$ bis $R_8$ wie im Anspruch 1 definiert sind, Verbindungen der Formel Ia reduziert, und die erhaltenen Verbindungen der Formel I in Form der freien Basen oder in Säureadditionssalzform gewinnt.

2. Verfahren gemäss Anspruch 1, dadurch gekennzeichnet, dass man Verbindungen der Formel I herstellt, worin

| | |
|---|---|
| $R_1$ und $R_2$ | je für Hydroxy stehen oder zusammen eine Oxogruppe bilden, |
| $R_3$ | Difluormethyl, Trifluormethyl, Chlordifluormethyl oder Pentafluorethyl bedeutet, |
| $R_4$, $R_8$ und $R_8$ | wie im Anspruch 1 definiert sind, |
| $R_7$ | für Methyl steht, und |
| $R_8$ | Wasserstoff bedeutet. |

3. Verfahren gemäss Anspruch 1, zur Herstellung des S-1-{3-[1-(Dimethylamino)ethyl]phenyl}-2,2,2-trifluor-1,1-ethandiols in Form der freien Base oder eines Säureadditionssalzes.

4. Verfahren gemäss Anspruch 1, zur Herstellung einer Verbindung aus der Gruppe bestehend aus
   1-{3-[1-(Dimethylamino)ethyl]phenyl}-2,2,2-trifluorethanon,
   1-{3-[1-(Dimethylamino)ethyl]phenyl}-2,2,2-trifluor-1,1-ethandiol,
   S-1-{3-[1-(Dimethylamino)ethyl]phenyl}-2,2,2-trifluorethanon,
   1-{3-[1-(Dimethylamino)ethyl]phenyl)}-2,2-difluorethanon,
   2-Chlor-1-{3-[1-(Dimethylamino)ethyl]phenyl}-2,2-difluorethanon,
   1-{3-[1-(Dimethylamino)ethyl]phenyl}-2,2,3,3,3-pentafluorpropanon,
   1-(5-Dimethylamino-5,6,7,8-tetrahydro-1-naphthalenyl)-2,2,2-trifluorethanon,
   1-(8-Dimethylamino-5,6,7,8-tetrahydro-2-naphthalenyl)-2,2,2-trifluorethanon und
   1-{3-[1-(Dimethylamino)ethyl]phenyl}-2,2,2-trifluorethanol,

   in Form der freien Base oder eines Säureadditionssalzes.

5. Verfahren zur Herstellung einer pharmazeutischen Zusammensetzung, enthaltend eine Verbindung der Formel I in Form der freien Base oder eines Säureadditionssalzes, dadurch gekennzeichnet, dass man die Verbindung der Formel I mit einem pharmazeutischen Hilfs- oder Trägerstoff vermischt.

## Claims

### Claims for the following Contracting States : AT, BE, CH, LI, DE, DK, FR, GB, IT, LU, NL, SE

1. A compound of formula I

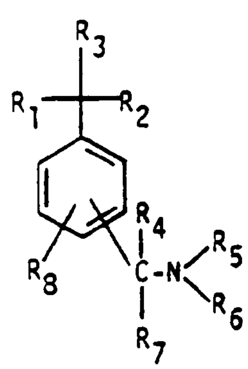

(I)

wherein

| | |
|---|---|
| $R_1$ | is hydrogen or hydroxy, |
| $R_2$ | is hydroxy or together with $R_1$ forms an oxo group, |
| $R_3$ | is $(C_{1-4})$alkyl substituted by 1 to 5 halogen atoms, |
| $R_4$ | is hydrogen or $(C_{1-4})$alkyl, |
| $R_5$ and $R_6$ | independently are $(C_{1-4})$alkyl, |
| $R_7$ | is $(C_{1-4})$alkyl and |

$R_8$ is hydrogen or $(C_{1-4})$alkyl, or is in ortho position to the dialkylaminoalkyl group and together with $R_7$ is $-(CH_2)_n-$ wherein n is 2, 3 or 4,

in free base or acid addition salt form.

2. A compound according to claim 1, wherein

$R_1$ and $R_2$ are hydroxy or together form an oxo group,

$R_3$ is difluoromethyl, trifluoromethyl, chlorodifluoromethyl or pentafluoroethyl,

$R_4$, $R_5$ and $R_5$ are as defined in claim 1,

$R_7$ is methyl, and

$R_8$ is hydrogen.

3. The S-1-{3-[1-(dimethylamino)ethyl]phenyl}-2,2,2-trifluoro-1,1-ethanediol in free base or acid addtion salt form.

4. A compound according to claim 1, from the group consisting of
1-{3-[1-(dimethylamino)ethyl]phenyl)-2,2,2-trifluoroethanone,
1-{3-[1-(dimethylamino)ethyllphenyl}-2,2,2-trifluoro-1,1-ethanediol,
S-1-{3-[1-(dimethylamino)ethyl]phenyl}-2,2,2-trifluoroethanone,
1-{3-[1-(dimethylamino)ethyl]phenyl}-2,2-difluoroethanone,
2-chloro-1-{3-[1-(dimethylamino)ethyl]phenyl}-2,2-difluoroethanone,
1-{3-[1-(dimethylamino)ethyl]phenyl}-2,2,3,3,3-pentafluoropropanone,
1-(5-dimethylamino-5,6,7,8-tetrahydro-1-naphthalenyl)-2,2,2-trifluoroethanone
1-(8-dimethylamino-5,6,7,8-tetrahydro-2-naphthalenyl)-2,2,2-trifluoroethanone and
1-{3-[1-(dimethylamino)ethyl]phenyl}-2,2,2-trifluoroethanol,
in free base or acid addition salt form.

5. A process for the production of a compound according to claim 1, characterized in that
a) for the production of compounds of formula Ia,

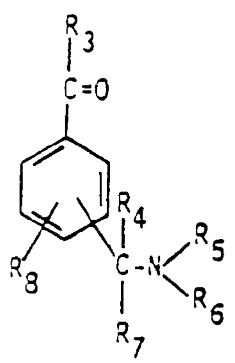

(Ia)

wherein $R_3$ to $R_8$ are as defined in claim 1, substituting the halogen in compounds of formula II,

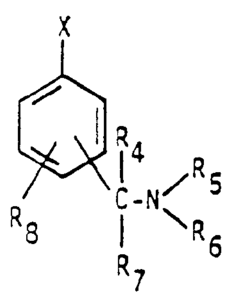

(II)

wherein $R_4$ to $R_5$ are as defined in claim 1 and X is chlorine, bromine or iodine, by a radical $-COR_3$, or
b) for the production of compounds of formula Ib,

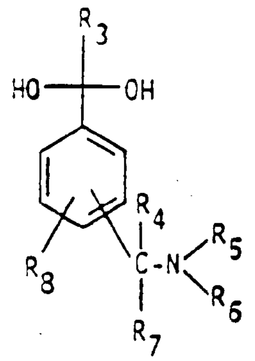

(Ib)

wherein $R_3$ to $R_8$ are as defined in claim 1, hydrating compounds of formula Ia, or
c) for the production of compounds of formula Ic,

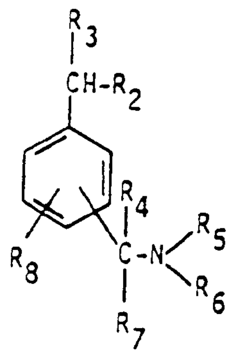

(Ic)

wherein $R_2$ to $R_8$ are as defined in claim 1, reducing compounds of formula Ia,
and recovering the thus obtained compounds of formula I in free base or acid addition salt form.

**6.** A compound according to any one of claims 1 to 4, in free or pharmacologically acceptable acid addition salt form, for use as a pharmaceutical.

**7.** A compound according to any one of claims 1 to 4, in free or pharmacologically acceptable acid addition salt form, for the treatment of senile dementia, Alzheimer's disease, Huntington's chorea, tardive dyskinesia, hyperkinesia, mania, acute confusional conditions, Down's syndrome, myasthenia gravis, Friedrich's ataxia and pain.

**8.** A pharmaceutical composition, comprising a compound according to any one of claims 1 to 4, in free or pharmaceutically acceptable acid addition salt form.

**Claims for the following Contracting States : ES, GR**

**1.** A process for the production of a compound of formula I,

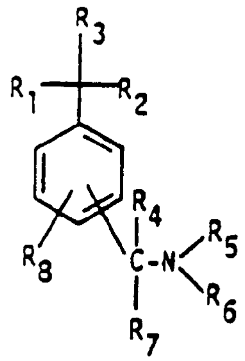

(I)

wherein

| | |
|---|---|
| $R_1$ | is hydrogen or hydroxy, |
| $R_2$ | is hydroxy or together with $R_1$ forms an oxo group, |
| $R_3$ | is $(C_{1-4})$alkyl substituted by 1 to 5 halogen atoms, |
| $R_4$ | is hydrogen or $(C_{1-4})$alkyl, |
| $R_5$ and $R_6$ | independently are $(C_{1-4})$alkyl, |
| $R_7$ | is $(C_{1-4})$alkyl and |
| $R_8$ | is hydrogen or $(C_1\text{-}_4)$alkyl, or is in ortho position to the dialkylaminoalkyl rest and together with $R_7$ is $-(CH_2)_n-$ wherein n is 2, 3 or 4, |

in free base or acid addition salt form, characterized in that

a) for the production of compounds of formula Ia,

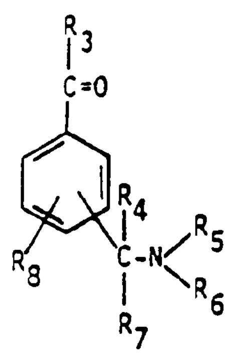

(Ia)

wherein $R_3$ to $R_8$ are as defined in claim 1, substituting the halogen in compounds of formula II,

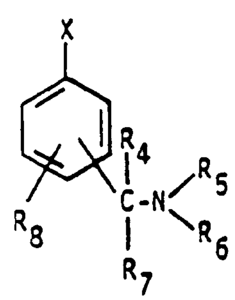

(II)

wherein $R_4$ to $R_8$ are as defined in claim 1 and X is chlorine, bromine or iodine, by a radical $-COR_3$, or

b) for the production of compounds of formula Ib,

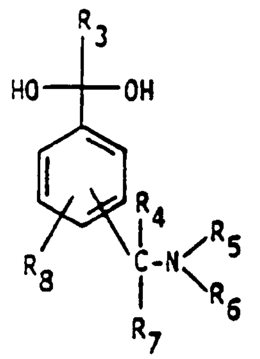

(Ib)

wherein $R_3$ to $R_8$ are as defined in claim 1, hydrating a compound of formula Ia, or
c) for the production of compounds of formula Ic,

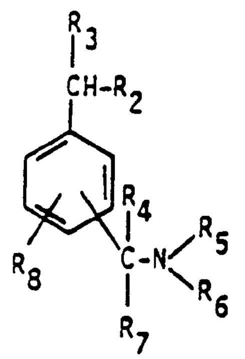

(Ic)

wherein $R_2$ to $R_8$ are as defined in claim 1, reducing compounds of formula Ia,
and recovering the thus obtained compounds of formula I in free base or acid addition salt form.

2. A process according to claim 1, characterized in that compounds of formula I are prepared, wherein

| | |
|---|---|
| $R_1$ and $R_2$ | are hydroxy or together form an oxo group, |
| $R_3$ | is difluoromethyl, trifluoromethyl, chlorodifluoromethyl or pentafluoroethyl, |
| $R_4$, $R_5$ and $R_5$ | are as defined in claim 1, |
| $R_7$ | is methyl, and |
| $R_8$ | is hydrogen. |

3. A process according to claim 1, for the preparation of S-1-{3-[1-(dimethylamino)ethyl]phenyl}-2,2,2-trifluoro-1,1-ethanediol in free base or acid addition salt form.

4. A process according to claim 1, for the preparation of a compound from the group consisting of
1-{3-[1-(dimethylamino)ethyl]phenyl}-2,2,2-trifluoroethanone
1-{3-[1-(dimethylamino)ethyl]phenyl}-2,2,2-trifluoro-1,1-ethanediol
S-1-{3-[1-(dimethylamino)ethyl]phenyl}-2,2,2-trifluoroethanone,
1-{3-[1-(dimethylamino)ethyl]phenyl}-2,2-difluoroethanone,
2-chloro-1-{3-[1-(dimethylamino)ethyl]phenyl}-2,2-difluoroethanone,
1-{3-[1-(dimethylamino)ethyl]phenyl}-2,2,3,3,3-pentafluoropropanone,
1-(5-dimethylamino-5,6,7,8-tetrahydro-1-naphthalenyl)-2,2,2-trifluoroethanone
1-(8-dimethylamino-5,6,7,8-tetrahydro-2-naphthalenyl)-2,2,2-trifluoroethanone and
1-{3-[1-(dimethylamino)ethyl]phenyl}-2,2,2-trifluoroethanol,
in free base or acid addition salt form.

5. A process for the production of a pharmaceutical composition comprising a compound of formula I as defined in claim 1 in free base or acid addition salt form, characterized in that the compound of formula I is mixed with a pharmaceutical adjuvant or carrier.

## Revendications

### Revendications pour les Etats contractants suivants : AT, BE, CH, LI, DE, DK, FR, GB, IT, LU, NL, SE

**1.** Un composé de formule I

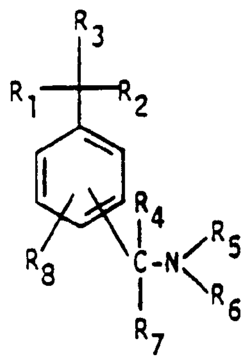

I

dans laquelle

| | |
|---|---|
| $R_1$ | signifie l'hydrogène ou un groupe hydroxy, |
| $R_2$ | signifie un groupe hydroxy ou forme un groupe oxo avec $R_1$, |
| $R_3$ | signifie un groupe alkyle en $C_1$-$C_4$ substitué par 1 à 5 atomes d'halogène, |
| $R_4$ | signifie l'hydrogène ou un groupe alkyle en $C_1$-$C_4$, |
| $R_5$ et $R_6$ | signifient, indépendamment l'un de l'autre, un groupe alkyle en $C_1$-$C_4$, |
| $R_7$ | signifie un groupe alkyle en $C_1$-$C_4$, et |
| $R_8$ | signifie l'hydrogène ou un groupe alkyle en $C_1$-$C_4$ ou forme, avec le reste dialkylaminoalkyle situé en position ortho et avec $R_7$, un groupe $-(CH_2)_n-$ où n signifie 2, 3 ou 4, |

sous forme de base libre ou d'un sel d'addition d'acide.

**2.** Un composé selon la revendication 1, dans lequel

| | |
|---|---|
| $R_1$ et $R_2$ | signifient chacun un groupe hydroxy ou forment ensemble un groupe oxo, |
| $R_3$ | signifie un groupe difluorométhyle, trifluorométhyle, chlorodifluorométhyle ou pentafluoroéthyle, |
| $R_4$, $R_6$ et $R_6$ | sont tels que définis à la revendication 1, |
| $R_7$ | signifie un groupe méthyle, et |
| $R_8$ | signifie l'hydrogène. |

**3.** Le S-1-{3-[1-(diméthylamino)éthyl]phényl}-2,2,2-trifluoro-1,1-éthanediol, sous forme de base libre ou d'un sel d'addition d'acide.

**4.** Un composé selon la revendication 1 choisi dans le groupe comprenant,
la 1-{3-[1-diméthylamino)éthyl]phényl}-2,2,2-trifluoroéthanone,
le 1-{3-[1-(diméthylamino)éthyl]phényl}-2,2,2-trifluoro-1,1-éthanediol,
la S-1-{3-[1-(diméthylamino)éthyl]phényl}-2,2,2-trifluoroéthanone,
la 1-{3-[1-(diméthylamino)éthyl]phényl}-2,2-difluoroéthanone,
la 2-chloro-1-{3-[1-(diméthylamino)éthyl]phényl}-2,2-difluoroéthanone,
la 1-{3-[1-(diméthylamino)éthyl]phényl}-2,2,3,3,3-pentafluoropropanone,
la 1-(5-diméthylamino-5,6,7,8-tétrahydro-1-naphtalényl)2,2,2-trifluoroéthanone,
la 1-(8-diméthylamino-5,6,7,8-tétrahydro-2-naphtalényl)-2,2,2-trifluoroéthanone, et
le 1-{3-[1-(diméthylamino)éthyl]phényl}-2,2,2-trifluoroéthanol,
sous forme de base libre ou d'un sel d'addition d'acide.

**5.** Un procédé de préparation d'un composé selon la revendication 1, caractérisé en ce que
a) pour la préparation des composés de formule Ia

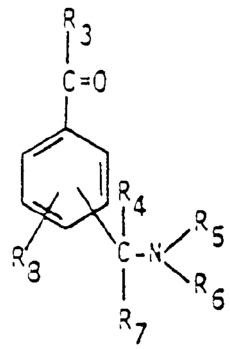

(I a)

où $R_3$ à $R_8$ sont tels que définis à la revendication 1, dans les composés de formule II

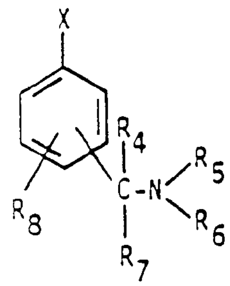

(II)

où $R_4$ à $R_8$ sont tels que définis à la revendication 1 et X signifie le chlore, le brome ou l'iode, on substitue l'halogène par un reste $-COR_3$, ou

b) pour la préparation des composés de formule Ib

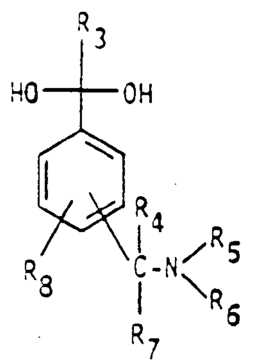

(I b)

où $R_3$ à $R_8$ sont tels que définis à la revendication 1, on hydrate les composés de formule Ia, ou

c) pour la préparation des composés de formule Ic

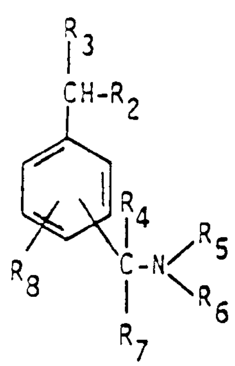

(I c)

où $R_2$ à $R_8$ sont tels que définis à la revendication 1, on réduit les composés de formule Ia,
et on récupère les composés de formule I obtenus, sous forme de base libre ou d'un sel d'addition d'acide.

**6.** Un composé selon l'une quelconque des revendications 1 à 4, sous forme libre ou sous forme d'un sel d'addition d'acide pharmacologiquement acceptable, pour l'utilisation comme médicament.

**7.** Un composé selon l'une quelconque des revendications 1 à 4, sous forme libre ou sous forme d'un sel d'addition d'acide pharmacologiquement acceptable, pour le traitement de la démence sénile, de la maladie de Alzheimer, de la chorée de Huntington, de la diskinésie tardive, de l'hyperkinésie, de la manie, des états confusionnels aigus, du syndrome de Down, de la myasthénie grave, de l'ataxie de Friedrich et des douleurs.

**8.** Une composition pharmaceutique, caractérisée en ce qu'elle contient un composé selon l'une quelconque des revendications 1 à 4, sous forme libre ou sous forme d'un sel d'addition d'acide pharmaceutiquement acceptable.

**Revendications pour les Etats contractants suivants : ES, GR**

**1.** Un procédé de préparation d'un composé de formule I

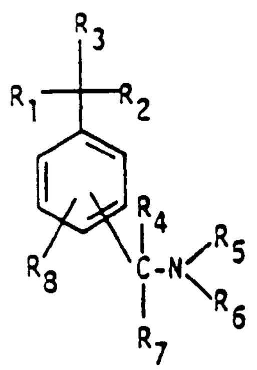

I

dans laquelle

| | |
|---|---|
| $R_1$ | signifie l'hydrogène ou un groupe hydroxy, |
| $R_2$ | signifie un groupe hydroxy ou forme un groupe oxo avec $R_1$, |
| $R_3$ | signifie un groupe alkyle en $C_1$-$C_4$ substitué par 1 à 5 atomes d'halogène, |
| $R_4$ | signifie l'hydrogène ou un groupe alkyle en $C_1$-$C_4$, |
| $R_5$ et $R_8$ | signifient, indépendamment l'un de l'autre, un groupe alkyle en $C_1$-$C_4$, |
| $R_7$ | signifie un groupe alkyle en $C_1$-$C_4$, et |
| $R_8$ | signifie l'hydrogène ou un groupe alkyle en $C_1$-$C_4$ ou forme, avec le reste dialkylaminoalkyle situé en position ortho et avec $R_7$, un groupe $-(CH_2)_n-$ où n signifie 2, 3 ou 4, |

sous forme de base libre ou d'un sel d'addition d'acide,
caractérisé en ce que

a) pour la préparation des composés de formule Ia

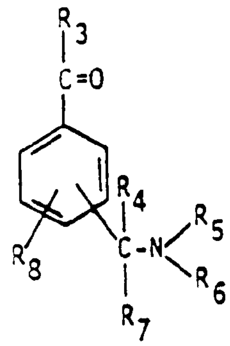

Ia

où $R_3$ à $R_8$ sont tels que définis à la revendication 1, dans les composés de formule II

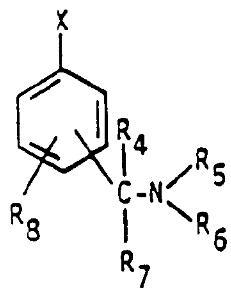

II

où $R_4$ à $R_8$ sont tels que définis à la revendication 1 et X signifie le chlore, le brome ou l'iode, on substitue l'halogène par un reste $-COR_3$, ou

b) pour la préparation des composés de formule Ib

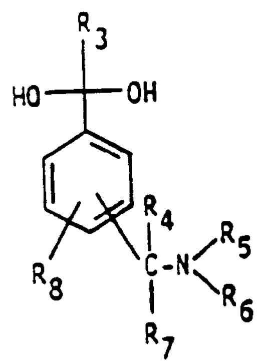

Ib

où $R_3$ à $R_8$ sont tels que définis à la revendication 1, on hydrate les composés de formule Ia, ou

c) pour la préparation des composés de formule Ic

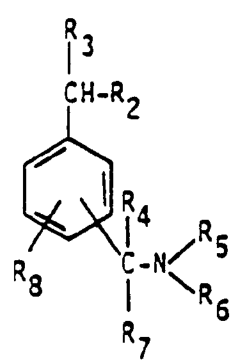

Ic

où $R_2$ à $R_8$ sont tels que définis à la revendication 1, on réduit les composés de formule Ia, et on récupère les composés de formule I obtenus, sous forme de base libre ou d'un sel d'addition d'acide.

2. Un procédé selon la revendication 1, caractérisé en ce qu'on prépare les composés de formule I, où

| | |
|---|---|
| $R_1$ et $R_2$ | signifient chacun un groupe hydroxy ou forment ensemble un groupe oxo, |
| $R_3$ | signifie un groupe difluorométhyle, trifluorométhyle, chlorodifluorométhyle ou pentafluoroéthyle, |
| $R_4$ , $R_5$ et $R_8$ | sont tels que définis à la revendication 1, |
| $R_7$ | signifie un groupe méthyle, et |
| $R_8$ | signifie l'hydrogène. |

3. Un procédé selon la revendication 1, pour la préparation du S-1-{3-[1-(diméthylamino)éthyl]phényl}-2,2,2-trifluoro-1,1-éthandiol, sous forme de base libre ou d'un sel d'addition d'acide.

4. Un procédé selon la revendication 1, pour la préparation d'un composé choisi dans le groupe comprenant
   la 1-{3-[1-diméthylamino)éthyl]phényl}-2,2,2-trifluoroéthanone,
   le 1-{3-[1-(diméthylamino)éthyl]phényl}-2,2,2-trifluoro-1,1-éthanediol,
   la S-1-{3-[1-(diméthylamino)éthyl]phényl}-2,2,2-trifluoroéthanone,
   la 1-{3-[1-(diméthylamino)éthyl]phényl}-2,2-difluoroéthanone,
   la 2-chloro-1-{3-[1-(diméthylamino)éthyl]phényl}-2,2-difluoroéthanone,
   la 1-{3-[1-(diméthylamino)éthyl]phényl}-2,2,3,3,3-pentafluoropropanone,
   la 1-(5-diméthylamino-5,6,7,8-tétrahydro-1-naphtalényl)2,2,2-trifluoroéthanone,
   la 1-(8-diméthylamino-5,6,7,8-tétrahydro-2-naphtalényl)-2,2,2-trifluoroéthanone, et
   le 1-{3-[1-(diméthylamino)éthyl]phényl}-2,2,2-trifluoroéthanol,

   sous forme de base libre ou d'un sel d'addition d'acide.

5. Un procédé de préparation d'une composition pharmaceutique contenant un composé de formule I sous forme de base libre ou d'un sel d'addition d'acide, caractérisé en ce qu'on mélange le composé de formule I avec un excipient ou un support pharmaceutiquement acceptable.